# EUROPEAN PATENT APPLICATION

(11) **EP 4 813 374 A1**
(43) Date of publication of application: **30.09.2026**
(21) Application number: 25216827.3
(22) Date of filing: 19.11.2025
(51) Int. Cl.: A61K 9/20, A61K 9/28, A61K 31/47

(54) **FILM COATED TABLET FORMULATIONS COMPRISING IVACAFTOR**

(30) Priority: 24.03.2025 TR 2025003496
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); ERSOY, Elif, Istanbul (TR); OZTAS, Necla, Istanbul (TR)
(74) Representative: Genç Ilhan, Oznur

(57) **Abstract**

The present invention relates to a film coated tablet formulation comprising a solid dispersion of ivacaftor and at least one pharmaceutical excipient wherein ivacaftor has a d (0.9) particle size between 5 µm and 25 µm. The present invention also relates to a simple, rapid, cost effective, time-saving and industrially convenient method of preparing the formulation.

## Description

### Field of Invention

The present invention relates to a film coated tablet formulation comprising a solid dispersion of ivacaftor and at least one pharmaceutical excipient wherein ivacaftor has a d (0.9) particle size between 5 µm and 25 µm. The present invention also relates to a simple, rapid, cost effective, time-saving and industrially convenient method of preparing the formulation.

### Background of the Invention

Ivacaftor, also known as N-(2,4-di-tert-butyl-5-hydroxyphenyl)-1,4-dihydro-4-oxoquinoline-3-carboxamide, having the following Formula (1):

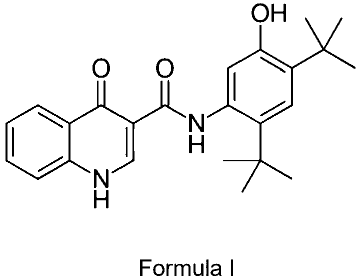

Ivacaftor is a white to off-white powder that is practically insoluble in water (<0.05 microgram/mL). Ivacaftor has low water solubility, very lipophilic and bioavailability of Ivacaftor is significantly enhanced when co-administered with food. Due to poor aqueous solubility, extensive formulation efforts were required and resulted in a spray-dried dispersion of Ivacaftor suitable for oral administration. Ivacaftor was approved by FDA and marketed by vertex pharma for the treatment of cystic fibrosis under the brand name KALYDECO^{®} in the form of 150 mg oral tablets. Kalydeco^{®} is indicated for the treatment of cystic fibrosis in patients age 6 years and older who have a G55ID mutation in the CFTR (cystic fibrosis transmembrane conductance regulator) gene.

Ivacaftor is a potentiator of the CFTR protein. The CFTR protein is a chloride channel present at the surface of epithelial cells in multiple organs. Ivacaftor facilitates increased chloride transport by potentiating the channel-open probability (or gating) of the CFTR protein.

Accordingly, there is a need for stable, bioavailable and high solubility pharmaceutical compositions of Ivacaftor useful for treating patients suffering from cystic fibrosis.

The patent IN20150262814 discloses to novel compositions of Ivacaftor. More specifically relates to unit solid oral compositions of crystalline Ivacaftor and one or more pharmaceutical excipients, wherein the particle size of Ivacaftor is of d(0.9) less than about 20 microns.

The patent US2015010628A1 discloses a solid dispersion of N-[2,4-Bis(1,1-dimethylethyl)-5-hydroxyphenyl]-1,4-dihydro-4-oxoquinoline-3-carboxamide including formulations of the solid dispersions into powders, granules and mini-tablets, methods for manufacturing and processing the powders and mini-tablets and methods for treating cystic fibrosis employing the pharmaceutical composition.

It is known that the solubility of ivacaftor in the prior art is poor and there are patent applications written for this. However, there is still a need for a formulation with both high stability and high solubility. In the present invention, a formulation with both high stability and high solubility was obtained by using a solid dispersion of ivacaftor and a particle size of d(0.9) between 5 µm and 25 µm.

### Detailed description of the Invention

The main object of the present invention is to provide a film-coated tablet formulation comprising a homogeneous ivacaftor with high solubility, the desired dissolution profile and high stability.

Another object of the present invention is to provide a preparation process of the film coated tablet formulation comprising ivacaftor which is simple and cost-effective process.

Ivacaftor has a low solubility in water, which hinders its absorption in the gastrointestinal tract. The solid dispersion formulation of Ivacaftor allows the drug to disperse in the hydrophobic matrix, offering more surface area for solubility. At the same time, a particle size as small as 5 µm to 25 µm provides a larger surface area for solubility of the drug. This is critical for drugs with low solubility because the faster the drug dissolves, the faster it is absorbed and passes into the bloodstream. The larger surface area improves solubility. The use of Ivacaftor's solid dispersion also provides higher stability than conventional ivacaftor formulations. The small particle size allows a better homogeneity to be achieved with the excipients used with the drug during the formulation process. In conclusion, our formulation using ivacaftor solid dispersion and d (0.9) particle size between 5 µm and 25 µm resulted in a homogeneous formulation with the desired dissolution profile and high stability.

According to this embodiment of the invention, a film coated tablet formulation comprising a solid dispersion of ivacaftor and at least one pharmaceutical excipient wherein ivacaftor has a d (0.9) particle size between 5 µm and 25 µm.

According to this embodiment of the invention, the solid dispersion comprises ivacaftor, hypromellose acetate succinate and sodium lauryl sulfate.

According to this embodiment of the invention, the solid dispersion comprises between 70.0% and 80.0% ivacaftor by weight in the dispersion, between 20.0% and 25.0 % of hypromellose acetate succinate by weight in the dispersion, and 0.1% and 1.0% of sodium lauryl sulfate by weight in the dispersion.

According to this embodiment of the invention, the amount of a solid dispersion of ivacaftor is between 25.0% and 45.0% by weight in the total formulation.

According to this embodiment of the present invention, the film coating tablet formulation comprises at least one pharmaceutically acceptable excipient which is selected from the group comprising fillers, disintegrants, surfactants, glidants, lubricants, film coating agents or a mixture thereof.

Suitable fillers are selected from the group comprising lactose monohydrate, microcrystalline cellulose, mannitol, lactose, dibasic calcium phosphate, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or a mixture thereof.

According to this embodiment of the present invention, the filler is lactose monohydrate or microcrystalline cellulose or mannitol or mixture thereof.

According to this embodiment of the present invention, the amount of filler is between 40.0% and 65.0% by weight of the total formulation, preferably, it is between 45.0% and 60.0% by weight of the total formulation. More preferably, the amount of filler is between 48.0% and 68.0% by weight of the total formulation.

Suitable disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to this embodiment of the present invention, the disintegrant is croscarmellose sodium.

According to this embodiment of the present invention, the amount of disintegrant is between 1.0% and 10.0% by weight of the total formulation, preferably it is between 3.0% and 7.0% by weight of the total formulation.

Suitable surfactants are selected from the group comprising sodium lauryl sulfate, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, poloxamers, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

According to this embodiment of the present invention, the surfactant is sodium lauryl sulfate.

According to this embodiment of the present invention, the amount of surfactant is between 0.01% and 5.0% by weight of the total formulation, preferably, it is between 0.1% and 2.0% by weight of the total formulation.

Suitable lubricants are selected from the group comprising sodium stearyl fumarate, glyceryl behenate, fatty acid, stearic acid, magnesium stearate, calcium stearate, sodium stearate, stearic acid, glyceryl monostearate, hydrogenated oils (i.e. hydrogenated vegetable oil), polyethylene glycol, fatty alcohol, fatty acid ester, glyceryl behenate, mineral oil, vegetable oil, leucine, sodium benzoate, or a mixture thereof.

According to this embodiment of the present invention, the lubricant is sodium stearyl fumarate or glyceryl behenate or mixture thereof.

According to this embodiment of the present invention, the amount of lubricant is between 0.01% and 8.0% by weight of the total formulation, preferably, it is between 0.1% and 5.0% by weight of the total formulation.

Suitable glidants are selected from the group comprising colloidal silicon dioxide, magnesium oxide, magnesium silicate, hydrophobic colloidal silica, calcium phosphate, powdered cellulose or a mixture thereof.

According to this embodiment of the present invention, the glidant is colloidal silicon dioxide.

According to this embodiment of the present invention, the amount of glidant is between 0.01% and 8.0% by weight of the total formulation, preferably, it is between 0.1% and 5.0% by weight of the total formulation.

Suitable film coating agents are selected from the group comprising polyvinyl alcohol (PVA), titanium dioxide, macrogol (PEG 3350), Indigo carmine aluminium lake, talc, carnauba wax, hydroxypropylmethylcellulose, polyethylene glycol (PEG), polyvinyl alcohol-polyethylene glycol copolymers, lecithin, polyvinylprolidone-vinyl acetate copolymer (PVP-VA), pigments, dyes, iron oxide or a mixture thereof.

According to this embodiment of the present invention, the amount of film coating agent is between 1.0% and 8.0% by weight of the total formulation.

In the present invention, the tablet comprises film coating to protect the composition against the moisture and light to maintain the stability.

A process for preparing a film coated tablet formulation comprising ivacaftor comprises the following steps;
a) weighing the solid dispersion of ivacaftor, fillers, disintegrant, surfactant and glidant and mixing,
b) sieving the mixture through a sieve,
c) finally, sieving the lubricant through a sieve and adding it to the mixture.

### Example 1;

| **Ingredients** | **%by weight** |
|---|---|
| Solid dispersion of ivacaftor | 25-45 |
| Lactose monohydrate 11SD (Supertab) | 10-40 |
| Microcrystalline cellulose 200 | 15-40 |
| Croscarmellose sodium | 1-10 |
| Sodium lauryl sulfate | 0.01-5 |
| Colloidal silicon dioxide | 0.01-8 |
| Sodium stearyl fumarate | 0.1-8 |
| **TOTAL CORE TABLET WEIGHT** | **92-99** |
| OPARDY II 85F505345 | 1-8 |
| - POLYVINYL ALCOHOL- PART. HYDROLYZED (USP, FCC, PhEur, JPE, ChP, GB) | |
| -TITANIUM DIOXIDE (USP, FCC, PhEur, JP, JSFA, ChP, GB) | |
| -MACROGOL/ PEG (USP, FCC, PhEur, JECFA, JP) | |
| -TALK (USP, FCC, PhEur, JP, JECFA, ChP) | |
| -FD&C BLUE #2/ INDIGO CARMINE ALUMINIUM LAKE (JECFA, JP MO, GB) | |
| -CARNAUBA WAX (NF, PhEur, JP, ChP, FCC) | |
| **TOTAL COATED TABLET WEIGHT** | **100** |

A process for Example 1;
a) weighing solid dispersion of ivacaftor, lactose monohydrate 11SD, microcrystalline cellulose 200, croscarmellose sodium, sodium lauryl sulfate, colloidal silicon dioxide and mixing for 10 minutes,
b) sieving the mixture through a 0.85 mm sieve,
c) finally, sieving sodium stearyl fumarate through a 0.425 mm sieve and adding to the mixture.

### Example 2;

| **Ingredients** | **%by weight** |
|---|---|
| Solid dispersion of ivacaftor | 25-45 |
| Lactose monohydrate 11SD (Supertab) | 46-65 |
| Croscarmellose sodium | 1-10 |
| Sodium lauryl sulfate | 0.01-5 |
| Colloidal silicon dioxide | 0.01-5 |
| Sodium stearyl fumarate | 0.01-5 |
| **TOTAL CORE TABLET WEIGHT** | **92-99** |
| OPARDY II 85F505345 | 1-8 |
| - POLYVINYL ALCOHOL- PART. HYDROLYZED (USP, FCC, PhEur, JPE, ChP, GB) | |
| -TITANIUM DIOXIDE (USP, FCC, PhEur, JP, JSFA, ChP, GB) | |
| -MACROGOL/ PEG (USP, FCC, PhEur, JECFA, JP) | |
| -TALK (USP, FCC, PhEur, JP, JECFA, ChP) | |
| -FD&C BLUE #2/ INDIGO CARMINE ALUMINIUM LAKE (JECFA, JP MO, GB) | |
| -CARNAUBA WAX (NF, PhEur, JP, ChP, FCC) | |
| **TOTAL COATED TABLET WEIGHT** | **100** |

A process for Example 2;
a) weighing solid dispersion of ivacaftor, lactose monohydrate 11SD, croscarmellose sodium, sodium lauryl sulfate, colloidal silicon dioxide and mixing for 10 minutes,
b) sieving the mixture through a 0.85 mm sieve,
c) finally, sieving sodium stearyl fumarate through a 0.425 mm sieve and adding to the mixture.

### Example 3;

| **Ingredients** | **%by weight** |
|---|---|
| Solid dispersion of ivacaftor | 25-45 |
| Lactose monohydrate 11SD (Supertab) | 20-40 |
| Microcrystalline cellulose 200 | 10-30 |
| Croscarmellose sodium | 1-10 |
| Sodium lauryl sulfate | 0.01-5 |
| Colloidal silicon dioxide | 0.01-5 |
| Glyceryl behenate | 0.5-8 |
| **TOTAL CORE TABLET WEIGHT** | **92-99** |
| OPARDY II 85F505345 | 1-8 |
| - POLYVINYL ALCOHOL- PART. HYDROLYZED (USP, FCC, PhEur, JPE, ChP, GB) | |
| -TITANIUM DIOXIDE (USP, FCC, PhEur, JP, JSFA, ChP, GB) | |
| -MACROGOL/ PEG (USP, FCC, PhEur, JECFA, JP) | |
| -TALK (USP, FCC, PhEur, JP, JECFA, ChP) | |
| -FD&C BLUE #2/ INDIGO CARMINE ALUMINIUM LAKE (JECFA, JP MO, GB) | |
| -CARNAUBA WAX (NF, PhEur, JP, ChP, FCC) | |
| **TOTAL COATED TABLET WEIGHT** | **100** |

A process for Example 3;
a) weighing solid dispersion of ivacaftor, lactose monohydrate 11SD, microcrystalline cellulose 200, croscarmellose sodium, sodium lauryl sulfate, colloidal silicon dioxide and mixing for 10 minutes,
b) sieving the mixture through a 0.85 mm sieve,
c) finally, sieving glyceryl behenate through a 0.425 mm sieve and adding to the mixture.

### Example 4;

| **Ingredients** | **%by weight** |
|---|---|
| Solid dispersion of ivacaftor | 25-45 |
| Lactose monohydrate 11SD (Supertab) | 45-65 |
| Mannitol | 1-20 |
| Microcrystalline cellulose 200 | 10-30 |
| Croscarmellose sodium | 1-10 |
| Sodium lauryl sulfate | 0.01-5 |
| Colloidal silicon dioxide | 0.5-8 |
| Sodium stearyl fumarate | 0.5-8 |
| **TOTAL CORE TABLET WEIGHT** | **92-99** |
| OPADRY II 85F505345 | 1-8 |
| - POLYVINYL ALCOHOL- PART. HYDROLYZED (USP, FCC, PhEur, JPE, ChP, GB) | |
| -TITANIUM DIOXIDE (USP, FCC, PhEur, JP, JSFA, ChP, GB) | |
| -MACROGOL/ PEG (USP, FCC, PhEur, JECFA, JP) | |
| -TALK (USP, FCC, PhEur, JP, JECFA, ChP) | |
| -FD&C BLUE #2/ INDIGO CARMINE ALUMINIUM LAKE (JECFA, JP MO, GB) | |
| -CARNAUBA WAX (NF, PhEur, JP, ChP, FCC) | |
| **TOTAL COATED TABLET WEIGHT** | **100** |

A process for Example 4;
a) weighing solid dispersion of ivacaftor, lactose monohydrate 11SD, mannitol, microcrystalline cellulose 200, croscarmellose sodium, sodium lauryl sulfate, colloidal silicon dioxide and mixing for 10 minutes,
b) sieving the mixture through a 0.85 mm sieve,
c) finally, sieving sodium stearyl fumarate through a 0.425 mm sieve and adding to the mixture.

### Example 5; A solid dispersion

| **Solid dispersion** | **% by weight** |
|---|---|
| Ivacaftor | 76 |
| Hypromellose acetate succinate | 23.5 |
| Sodium lauryl sulfate | 0.5 |
| **Total** | **100** |

A process for Example 5;
a) Weighing ivacaftor, hypromellose acetate succinate, sodium lauryl sulfate and mixing for 10 minutes,
b) Dissolving the mixture in water and drying at 50°C in a fluidized bed dryer,
c) Sieving the mixture through a 1mm square sieve.

## Claims

1. A film coated tablet formulation comprising a solid dispersion of ivacaftor and at least one pharmaceutical excipient wherein ivacaftor has a d (0.9) particle size between 5 µm and 25 µm.

2. The film coated tablet formulation according to claim 1, wherein the amount of a solid dispersion of ivacaftor is between 25.0% and 45.0% by weight in the total formulation.

3. The film coated tablet formulation according to claim 1, wherein the film coating tablet comprises at least one pharmaceutically acceptable excipient which is selected from the group comprising fillers, disintegrants, surfactants, glidants, lubricants, film coating agents or a mixture thereof.

4. The film coated tablet formulation according to claim 3, wherein fillers are selected from the group comprising lactose monohydrate, microcrystalline cellulose, mannitol, lactose, dibasic calcium phosphate, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or a mixture thereof.

5. The film coated tablet formulation according to claim 3 or 4, wherein the filler is lactose monohydrate or microcrystalline cellulose or mannitol or mixture thereof.

6. The film coated tablet formulation according to claim 4 or 5, wherein the amount of filler is between 40.0% and 65.0% by weight of the total formulation, preferably, it is between 45.0% and 60.0% by weight of the total formulation.

7. The film coated tablet formulation according to claim 3, wherein disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

8. The film coated tablet formulation according to claim 3 or 7, wherein the disintegrant is croscarmellose sodium.

9. The film coated tablet formulation according to claim 7 or 8, wherein the amount of disintegrant is between 1.0% and 10.0% by weight of the total formulation, preferably it is between 3.0% and 7.0% by weight of the total formulation.

10. The film coated tablet formulation according to claim 3, wherein surfactants are selected from the group comprising sodium lauryl sulfate, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, poloxamers, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

11. The film coated tablet formulation according to claim 3 or 10, wherein the surfactant is sodium lauryl sulfate.

12. The film coated tablet formulation according to claim 10 or 11, wherein the amount of surfactant is between 0.01% and 5.0% by weight of the total formulation, preferably, it is between 0.1% and 2.0% by weight of the total formulation.

13. The film coated tablet formulation according to claim 3, wherein lubricants are selected from the group comprising sodium stearyl fumarate, glyceryl behenate, fatty acid, stearic acid, magnesium stearate, calcium stearate, sodium stearate, stearic acid, glyceryl monostearate, hydrogenated oils (i.e. hydrogenated vegetable oil), polyethylene glycol, fatty alcohol, fatty acid ester, glyceryl behenate, mineral oil, vegetable oil, leucine, sodium benzoate, or a mixture thereof.

14. The film coated tablet formulation according to claim 3 or 13, wherein the lubricant is sodium stearyl fumarate or glyceryl behenate or mixture thereof.

15. The film coated tablet formulation according to claim 13 or 14, wherein the amount of lubricant is between 0.01% and 8.0% by weight of the total formulation, preferably, it is between 0.1% and 5.0% by weight of the total formulation.
